Europäisches Patentamt

European Patent Office

Office européen des brevets

(11) Veröffentlichungsnummer: **0 486 717 A1**

## EUROPÄISCHE PATENTANMELDUNG

(21) Anmeldenummer: **90122194.5**

(51) Int. Cl.⁵: **A61B 6/00**

(22) Anmeldetag: **20.11.90**

(43) Veröffentlichungstag der Anmeldung:
**27.05.92 Patentblatt 92/22**

(84) Benannte Vertragsstaaten:
**DE FR GB**

(71) Anmelder: **SIEMENS AKTIENGESELLSCHAFT**
**Wittelsbacherplatz 2**
**W-8000 München 2(DE)**

(72) Erfinder: **Engel, Thomas, Dipl.-Ing.**
**Breslauer Strasse 2**
**W-8520 Erlangen(DE)**
Erfinder: **Höbel, Peter, Dipl.-Ing. FH**
**Im Föhrenwald 3**
**W-8520 Erlangen(DE)**
Erfinder: **Schwarzmann, Kurt, Dipl.-Ing.**
**Beethovenstrasse 8b**
**W-8552 Höchstadt(DE)**
Erfinder: **Ussmüller, Jürgen, Dipl.-Ing. FH**
**Am Kreuzweiher 11**
**W-8602 Stegaurach(DE)**

(54) **Medizinische Diagnostikanlage.**

(57) Es soll eine medizinische Diagnostikanlage, insbesondere eine Röntgen-Angiographieanlage, mit mehreren Komponenten (1 bis 4, 15) hinsichtlich der Bedienung und Überwachung gegenüber dem Stand der Technik vereinfacht werden. Ferner soll der Datenfluß, z.B. Parameter für eine optimale Bildqualität, koordiniert werden können.

Es ist ein zentraler Arbeitsplatz (6) vorhanden, der eine Bedienfläche (10) aufweist, die zur zentralen Bedienung aller Komponenten (1 bis 4, 15) dient. Auch die Überwachung der Komponenten (1 bis 4, 15) und die Erfassung, Speicherung und Verteilung der Patienteninformationen erfolgen an dem zentralen Arbeitsplatz (6) mit Hilfe von Monitoren (7, 8, 9).

Es sind medizinische Diagnostikanlagen mit einer Vielzahl von Komponenten bekannt, bei denen den einzelnen Komponenten Bedienpulte für die Steuerung zugeordnet sind. So sind z.B. Röntgen-Angiographieanlagen bekannt, die aus einem Röntgenuntersuchungsgerät, einem Röntgengenerator, einem Kontrastmittelinjektor sowie Patientenüberwachungsgeräten, z.B. zur Aufnahme des EKG, zusammengesetzt sind. Bei einer medizinischen Diagnostikanlage dieser Art werden höchste Ansprüche an die Bildqualität gestellt. Ferner besteht der Wunsch nach einer möglichst einfachen und übersichtlichen Bedienung, damit das Untersuchungspersonal sich voll auf den Patienten konzentrieren kann. Die Vielzahl der Bedienpulte bei den bekannten Diagnostikanlagen erschwert den Umgang mit der Anlage und kann Anlaß für Fehler sein.

Der Erfindung liegt die Aufgabe zugrunde, eine medizinische Diagnostikanlage mit mehreren Komponenten hinsichtlich der Bedienung und der Überwachung, insbesondere des Patienten, d.h. der Erfassung von Patientendaten und Patientenparametern gegenüber dem Stand der Technik, zu vereinfachen und zu verbessern.

Diese Aufgabe ist erfindungsgemäß dadurch gelöst, daß ein zentraler Arbeitsplatz vorhanden ist, von dem aus die Komponenten bedienbar und überwachbar sind und der auch Patienteninformationen, z.B. das EKG, erfaßt und zentral zeitsynchron und/oder chronologisch wiedergibt. Bei der erfindungsgemäßen medizinischen Diagnostikanlage erfolgt die Bedienung und Überwachung der Komponenten sowie die Erfassung von Patienteninformationen zentral an einer einheitlichen Bedienoberfläche. Die Bedienorgane sind demgemäß nicht mehr komponenten- sondern anlagenbezogen. Die Bedienung selbst läuft weitgehend in Programmtechnik ab.

Diese Technik eignet sich besonders für die Röntgen-Angiographie, d.h. für Anlagen zur Untersuchung der Gefäße eines Patienten, insbesondere der Herzgefäße.

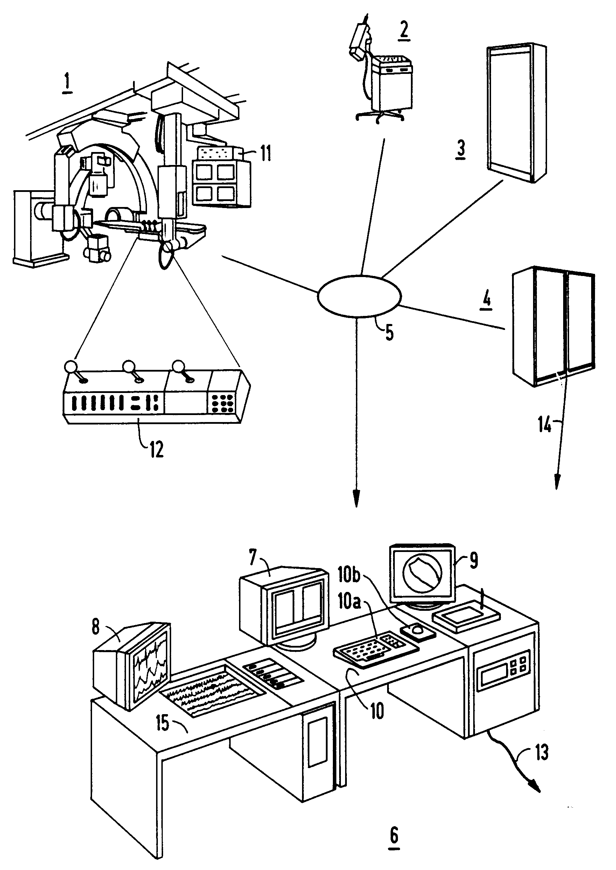

Die Erfindung ist nachfolgend anhand eines in der Zeichnung dargestellten Ausführungsbeispiels näher erläutert.

In der Zeichnung ist eine Röntgen-Angiographieanlage zur Herzuntersuchung dargestellt, die ein Zwei-Ebenen-Angiographiegerät 1 aufweist. Mit dem Angiographiegerät 1 kann der Patient im Herzbereich aus zwei zueinander senkrechten Richtungen durchstrahlt werden. Zur Darstellung der Blutgefäße des Patienten wird diesem Kontrastmittel injiziert, und zwar mit Hilfe eines Injektors 2. Zur Speisung der Röntgenröhren des Angiographiegerätes 1 ist ein Röntgengenerator 3 mit einem Hochspannungstransformator vorhanden. Ein digitales Bildsystem 4 dient zur digitalen Erfassung, Verarbeitung und Speicherung der erzeugten Röntgenbilder.

Die Komponenten 1 bis 4 sind über ein Netzwerk 5 zusammengeschaltet, über das Daten übertragen werden.

Am Netzwerk 5 ist ein zentraler Arbeitsplatz 6 mit Monitoren 7, 8, 9 und einer Bedienfläche 10 angeschlossen, der die Komponenten 1 bis 4 steuert und überwacht und auch zur Erfassung von Patienteninformationen dient.

Am zentralen Arbeitsplatz 6 werden die Parameter der Komponenten 1 bis 4 mit Hilfe der Bedienfläche 10 und der Monitore 7, 8, 9 eingestellt. So können z.B. die Tischplattenverschiebung zur optimalen Positionierung des Patienten am Angiographiegerät 1, die Parameter der Röntgenröhren der beiden Röntgensysteme des Angiographiegeräts 1 (mA, kV, t) sowie die Parameter des Injektors 2 eingestellt werden. Die jeweils eingestellten Werte werden auf den Monitoren 7, 8, 9 wiedergegeben und können dort überwacht werden.

Der Monitor 8 dient zur Wiedergabe physiologischer Meßwerte. So können z.B. die von einem EKG-Gerät 11 gelieferten Meßwerte über das Netzwerk 5 zeitsynchron zur Bildinformation an den Arbeitsplatz 6 übertragen und auf dem Monitor 8 wiedergegeben werden.

Der zentrale Arbeitsplatz 6 kann nicht nur zur Steuerung und Überwachung der Komponenten 1 bis 4 während des normalen Betriebes, sondern auch für Servicezwecke benutzt werden. Der Servicetechniker kann z.B. bestimmte Parameter für Servicezwecke von dort aus einstellen und ihre Einstellung überwachen.

Für die unmittelbare Bedienung von Komponenten können hierfür erforderliche Bedienelemente zusätzlich an der jeweiligen Komponente patientennah angeordnet sein. Ein Bedienpult 12 für diesen Zweck ist in Verbindung mit dem Angiographiegerät 1 dargestellt. Während am Bedienpult 12 die Bedienung über Schalter, Hebel u.dgl. erfolgt, erfolgt die Bedienung am zentralen Arbeitsplatz 6 auf der Bedienfläche 10 mit Hilfe einer Tastatur in Verbindung mit einem am Arbeitsplatz 6 vorhandenen zentralen Rechner 15. Dieser Rechner 15 ist in das Netzwerk 5 eingeschaltet und kann über eine Leitung 13 mit einem Klinikrechner zum Datenaustausch verbunden sein. Das digitale Bildsystem 4 kann über eine Leitung 14 an einem zentralen Bildarchivierungssystem angeschlossen sein.

Bei der beschriebenen Röntgen-Angiographieanlage ist die Vielzahl der Bedienelemente am Arbeitsplatz 6, und zwar auf dessen Bedienfläche 10 in Form einer optischen Zeigerbedienung, z.B. Mausbedienung 10a oder alphanumerischen Tastatur 10b, integriert. Diese Tastatur 10b steuert den Rechner 15. Von dort aus erfolgt die Bedienung der Komponenten 1 bis 4. Die Überwachung dieser Komponenten und die Erfassung physiologischer Daten erfolgt mit Hilfe der Monitore 7, 8, 9 ebenfalls zentral. Die Vielzahl der beim Stand der Technik vorhandenen Bedienorte ist demgemäß bei der beschriebenen Röntgen-Angiographieanlage beseitigt.

**Patentansprüche**

1. Medizinische Diagnostikanlage mit mehreren Komponenten (1 bis 4, 15) und einem zentralen Arbeitsplatz (6), von dem aus die Komponenten (1 bis 4, 15) bedienbar und überwachbar sind und der Patienteninformationen erfaßt.

2. Medizinische Diagnostikanlage nach Anspruch 1, bei der der zentrale Arbeitsplatz (6) zur Darstellung, Erfassung und Einstellung der Betriebsparameter der Komponenten (1 bis 4, 15) ausgebildet ist.

3. Medizinische Diagnostikanlage nach Anspruch 1 oder 2, bei der der zentrale Arbeitsplatz (6) einen Monitor (7, 9) zur Wiedergabe der eingestellten und von den Komponenten (1 bis 4, 15) übertragenen Parameter aufweist und ein Rechner (15) zur Steuerung des Datenflusses und der Datenwiedergabe vorhanden ist.

4. Medizinische Diagnostikanlage nach einem der Ansprüche 1 bis 3, bei der zusätzlich zum zentralen Arbeitsplatz (6) eine patientennahe Anlagenbedienung (12) vorhanden ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | FR-A-2641180 (GENERAL ELECTRIC CGR SA.) * Seite 5, Zeile 24 - Seite 11, Zeile 20; Figuren * | 1 | A61B6/00 |
| A | | 2-4 | |
| | --- | | |
| X | EP-A-66008 (SIEMENS AKTIENGESELLSCHAFT) * Seite 2, Zeile 6 - Seite 6, Zeile 16; Figuren * | 1 | |
| A | | 2, 3 | |
| | --- | | |
| X | FR-A-2178596 (SIEMENS AG) * Seite 2, Zeile 11 - Seite 4, Zeile 31; Figuren * | 1 | |
| A | | 2, 3 | |
| | --- | | |
| X | FR-A-2594321 (INGLESE ET AL.) * Seite 2, Zeile 6 - Seite 5, Zeile 21; Figur * | 1 | |
| A | | 2, 3 | |
| | ----- | | |

RECHERCHIERTE
SACHGEBIETE (Int. Cl.5 )

A61B

Der vorliegende Recherchenbericht wurde für alle Patentansprüche erstellt

| Recherchenort | Abschlußdatum der Recherche | Prüfer |
|---|---|---|
| DEN HAAG | 22 JULI 1991 | CHEN A.H. |